# EUROPEAN PATENT SPECIFICATION

(11) **EP 1 018 933 B1**
(45) Date of publication and mention of the grant of the patent: **16.01.2008**
(21) Application number: 98948547.9
(22) Date of filing: 25.09.1998
(51) Int. Cl.: A61B 5/00

(54) **DEVICE FOR ASSESSING PERFUSION FAILURE IN A PATIENT**
GERÄT ZUR BESTIMMUNG VON STÖRUNGEN DER PERFUSION EINES PATIENTENS
APPAREIL PERMETTANT D'EVALUER UNE DEFICIENCE DE L'IRRIGATION SANGUINE CHEZ UN PATIENT

(30) Priority: 29.09.1997 US 939591; 18.06.1998 US 99293; 24.09.1998 US
(43) Date of publication of application: 19.07.2000
(73) Proprietor: INSTITUTE OF CRITICAL CARE MEDICINE, Palm Springs, CA 92262 (US)
(72) Inventor: WEIL, Max, Harry, Northbrook, IL 60062 (US); TANG, Wanchun, Palm Desert, CA 92260 (US); BISERA, Jose, Camarillo, CA 93010 (US)
(74) Representative: Belcher, Simon James
(86) International application number: PCT/US1998/020118
(87) International publication number: WO 1999/016346

(56) References cited:
- WO-A-94/23645
- US-A- 4 890 619
- US-A- 5 368 027
- OGINO H ET AL: "REFLECTANCE PULSE OXIMETER MEASURING CENTAL SAO2 FROM MOUTH" PROCEEDINGS OF THE ANNUAL INTERNATIONAL CONFERENCE OF THE IEEE ENGINEERING IN MEDICINE AND BIOLOGY SOCIETY, BALTIMORE, NOV. 3 - 6, 1994, vol. 2, no. VOL. 16, 3 November 1994, page 914/915 XP000552397 SHEPPARD N F;EDEN M; KANTOR G (EDS )

## Description

The present invention relates generally to devices for assessing perfusion failure in a patient. More particularly, the invention relates to assessment of perfusion failure in a patient by measuring the localized partial pressure of carbon dioxide within the upper respiratory/digestive tract of a patient.

### BACKGROUND ART

Very low blood flow, or low "systemic perfusion," is typically due to low aortic pressure and can be caused by a number of factors, including hemorrhage, sepsis and cardiac arrest. When there is a reduced flow of blood from the heart, the body directs a higher portion of blood to critical organs, such as the brain, which will not survive long without a continuous supply of blood, while restricting the flow to less critical organs, such as the stomach and intestines, whose survival is not as threatened by a temporary large reduction in blood flow. Physicians commonly take advantage of this phenomenon by taking measurements in the stomach and intestine to assess perfusion failure.

Assessment of CO₂ concentration in the less critical organs. i.e.. those organs to which blood flow is reduced during perfusion failure, is useful in perfusion assessment. Carbon dioxide production, which is associated with metabolism, continues even during low blood flow. Because CO₂ is not rapidly carried away during low blood flow, the concentration of CO₂ increases, which in turn results in a decrease in pH and an increase in partial pressure of CO₂ (pCO₂) in the less critical organs. Therefore, perfusion failure is commonly assessed by measuring pH or pCO₂ at these sites, especially in the stomach and intestines. For examples of catheters used to assess pH or pCO₂ in the stomach or intestines, see, e.g., U.S. Patent Nos. 3,905,889; 4.016,863; 4,632,119; 4,643,192; 4,981,470; 5.105.812; 5.117.827; 5,174,290; 5,341,803: 5,411,022; 5,423,320; 5,456,251; and 5.788,631.

The measurement of pCO₂ to determine the extent of perfusion failure has commonly been done by threading a catheter through the nasal passage, past the epiglottis, through the esophagus, past the esophageal sphincter, and into the stomach, and sometimes through the stomach and into the intestines. Alternatively, measurement has been conducted in the colon, with a catheter being threaded through the anus. These procedures are obviously quite invasive and can cause harm and discomfort to a patient. Moreover, insertion of the catheter in this manner is also complex and time-consuming.

In U.S. Patent No. 5,579,763, applicants described the introduction of a catheter with a carbon dioxide sensor through the nasal or oral passage, past the epiglottis, and into the esophagus so that the catheter and sensor lay within the esophagus. This method can be used to accurately assess perfusion failure by measuring pCO₂ in the patient's esophagus of a patient, rather than in the stomach and/or intestine. Tests showed that measurements of pCO₂ in the esophagus are closely correlated with aortic pressure, and, furthermore, that measurements made in the esophagus are even more closely correlated to aortic pressure than measurements of CO₂ in the stomach. This procedure was advantageous in that the procedure's invasiveness was reduced and CO₂ generated by digestive fluids in the stomach did not affect measurements since the esophageal sphincter blocks such gas. However, the insertion of the catheter still constituted considerable invasion and thus risk of harm to the patient. Furthermore, extension of the catheter extended past the epiglottis exposed the patient to the risk of regurgitation of stomach contents including stomach acids.

There is a need for an even less invasive method to measure perfusion failure and to monitor the effectiveness of methods taken to increase perfusion, *e.g*., blood infusion or the like.

### DISCLOSURE OF THE INVENTION

Methods and devices are provided for assessing impairment of blood circulation in a patient, such as that in perfusion failure, by measurement of pCO₂ (partial pressure of carbon dioxide) in the upper digestive and/or respiratory tract of the patient. The method comprises introducing a carbon dioxide sensor into the upper digestive and/or respiratory tract of a patient, without passing the sensor down through or beyond the patient's epiglottis. Specifically, a carbon dioxide sensor is placed adjacent a mucosal surface within the upper digestive and/or respiratory tract, preferably within the patient's mouth or inside the patient's nose. By avoiding passage through the mouth into the throat and esophagus, discomfort is substantially avoided and the potential for injury minimized. Previously, the belief in the art was that increased partial pressure of carbon dioxide was a localized phenomenon during perfusion failure; however, applicants have now discovered that increases in tissue CO₂ occur throughout the body during perfusion failure, and the device of the invention is premised on this discovery.

Applicants prefer to introduce the carbon dioxide sensor sublingually, and preferably to one side of the frenulum. The invasiveness of such a technique is minimal, being substantially no more than in the use of an oral thermometer. The sensor preferably lies at the inner end of a holder that lies stably in the patient's mouth. The holder maintains the sensor in position, and also isolates the area immediately surrounding the mucosal surface contacted by the sensor from surrounding air flow that could carry away some CO₂ and result in an incorrect measurement. Preferably, the sensor is an optical CO₂ sensor. The output of the sensor can be detected by a device which electronically converts the sensor output to provide a CO₂ concentration value. The device can further sense the rate of change of CO₂ concentration with time to indicate the patient's condition.

Accordingly, in one aspect the invention features a device for assessing perfusion failure in a patient, where the device is composed of a carbon dioxide sensor means for detecting a partial pressure of carbon dioxide (pCO₂), the sensor means being adapted for lying adjacent a mucosal surface of the upper respiratory/digestive tract of a patient and measuring carbon dioxide at the mucosal surface: and an indicating means connected to the sensor means, wherein the indicating means indicates a degree of perfusion failure of the patient associated with the detected partial pressure of carbon dioxide. The device also includes an isolating means for inhibiting air flow around the mucosal surface in a region surrounding the sensor means.

In a preferred embodiment, the isolating means is a holder designed to fit within the mouth of the patient and hold the sensor in place adjacent the mucosal surface. The holder may be designed to contact the bottom of the tongue and the floor of the mouth of the patient, or to fit between the inside of a lip and gum of the patient. In another embodiment, the isolating means is a holder designed to fit within a nares of the patient and hold the sensor in place adjacent the mucosal surface.

In another preferred embodiment, the device includes a moisturizing means for supplying moisture to the mucosal surface adjacent the sensor.

A device for use with a pCO₂ sensor assembly for assessing perfusion failure of a patient, is composed of a sensor holder with a sublingual holder inner portion shaped to fit in the mouth of a patient under the patient's tongue, said holder forming at least one holder passage extending from said holder outer portion to said sublingual holder portion.

A method for assessing perfusion failure of a patient involves the steps of placing a carbon dioxide sensor adjacent a mucosal surface of an upper digestive/respiratory tract of a patient, and measuring a partial pressure of carbon dioxide at the mucosal surface. A partial pressure of carbon dioxide at the mucosal surface of the upper digestive/respiratory tract that is substantially greater than a normal partial pressure of carbon dioxide is indicative of perfusion failure in the patient. In preferred embodiments the mucosal surface is within the mouth or nose of the patient.

One advantage of the invention is that perfusion can be assessed in a patient in a minimally invasive manner, and with minimal discomfort or risk of harm to the patient.

Another advantage of the invention is that perfusion can be readily assessed in a patient suffering from perfusion failure associated with any of a variety of causes, including, but not limited to physical trauma, infection, hypothermia, cardiogenic shock (*e.g*., acute myocardial infarction, aneurysm, or arrhythmia), obstructive shock (*e.g*., pulmonary embolism), hypovolemic shock (*e.g*., due to hemorrhage or fluid depletion), and distributive shock (*e.g*., due to sepsis, exposure to toxins, or anaphylaxis). The sensitivity of the devices of the invention further allow for assessment of perfusion across a wide range of perfusion failure severity, thereby providing a means to accurately monitor the patient's condition.

Still another advantage of the invention is that the devices and methods can be readily adapted for use in alert, semi-conscious, or unconscious patients, and can be further adapted for accurate assessment of perfusion in a patient for a period lasting for only minutes to hours or days.

The novel features of the invention are set forth with particularity in the appended claims. The invention will be best understood from the following description when read in conjunction with the accompanying drawings.

### BRIEF DESCRIPTION OF DRAWINGS

Fig. 1 is a partial sectional view of the digestive system of a patient (including the nasal passage), and showing a previous sensor of applicant which is fully installed during a test.
Fig. 2 is an isometric view showing a sensor of the present invention as it is introduced into the mouth of a patient, for sublingual placement.
Fig. 3 is a sectional view showing the sensor of Fig. 2 fully installed in a patient's mouth.
Fig. 4 is a graph that includes a graph line showing variation in aortic pressure with time, and that also includes a graph line showing variation in sublingual pCO₂ measurement with time, during an experiment on a rat.
Fig. 5 is a sectional view of a sensor assembly and holder constructed in accordance with another embodiment of the invention, shown lying in a patient's mouth.
Fig. 6 is an inner isometric view of the holder of Fig 5.
Fig. 7 is an outer isometric view of the holder of Fig. 5.
Fig. 8 is a graph that includes graph lines showing sublingual response with and without the holder of Fig. 5.
Fig. 9 is an electrical block diagram of a circuit for processing data that includes the output of the CO₂ sensor of Fig. 5.
Fig. 10 is a chart that shows the logic of the circuit of Fig. 9.
Fig. I 1 is a top and outer isometric view of a holder of another embodiment of the invention.
Fig. 12 is a sectional view of the holder of Fig. 11, shown lying in a patient's mouth, with a sensor assembly in place.
Fig. 13 is a sectional view of a sensor assembly and holder of another embodiment of the invention, shown holding a sensor between a lip and teeth of a patient.
Fig. 14 is a front isometric view of the holder of Fig. 13.
Fig. 15 is a sectional view of a sensor assembly and holder of another embodiment of the invention, shown holding a sensor in the nose of a patient.
Fig. 16 is a sectional view of a sensor assembly and holder of another embodiment of the invention, where the holder can add moisture to the area of the sensor.
Fig. 17 is a graph showing CO₂ sensor drift with time in different environments.

### BEST MODE FOR CARRYING OUT THE INVENTION

### Definitions and nomenclature:

Before the present compounds, compositions and methods are disclosed and described, it is to be understood that this invention is not limited to sensor designs, measurement techniques, or the like, as such may vary. It is also to be understood that the terminology used herein is for the purpose of describing particular embodiments only and is not intended to be limiting.

It must be noted that, as used in the specification and the appended claims, the singular forms "a," "an" and "the" include plural referents unless the context clearly dictates otherwise.

The term "perfusion failure" as used herein is meant a reduction in blood flow associated with maldistribution of blood through the circulatory system and a reduction in blood flow to a less critical tissue(s) and/or organ(s) relative to blood flow in vital (critical) tissues and organs (*e.g*., the brain and heart). In general. "perfusion failure" is meant to encompass reduction in blood flow associated with a increase in pCO₂ of significantly or substantially above a pCO₂ associated with normal perfusion.

The term "measurement" as used herein refers to a single measurement or a series of measurements made over time, and which may be taken continuously or intermittently (*e.g*., at selected time intervals).

The term "sample fluid" as used herein refers to a liquid or gaseous material (*e.g*., vapor, mist, or gas) that may be analyzed using the sensors disclosed herein. Generally, "sample fluids" analyzed in the course of assessing perfusion failure will be a mixture of gas and fluid trapped within the area defined by the sensor holder walls and a mucosal surface with which the sensor holder is in contact.

The term "upper respiratory/digestive tract" as used herein means the region of the upper respiratory tract and digestive tract at the surface or and above the epiglottis. In general, the "upper respiratory/digestive tract" encompasses the nasal passages (including the nares and nasal cavities), the oral passage (including the mouth and spaces within the mouth such as the floor (*e.g*., sublingual area) and roof of the mouth (e.g., hard palate), the soft palate, the regions between the lips and gums, and the cheeks and gums), the nasopharynx, and the upper portion of the throat that extends to the top surface of and in the region of the epiglottis.

The term "oral-nasal cavity" as used herein means the region of the upper respiratory/digestive tract encompassing the nasal passages (including the nares and nasal cavities), the oral passage (including the mouth and spaces within the mouth such as the floor (*e.g*., sublingual area) and roof of the mouth (*e.g.*, hard palate), the soft palate, the regions between the lips and gums, and the cheeks and gums), and the nasopharynx.

The term "sublingual" as used herein refers to a region below or beneath the tongue.

The term "mucosal surface" as used herein refers to a surface of a mucous membrane containing or associated with mucus secreting glands, and which lines body passages, tubular structures, and organs. In general, "mucosal surface" is meant to refer to the surface of the membranes lining the digestive and respiratory tracts.

The term "adjacent" as used herein (*e.g*., "adjacent the mucosal surface") means near or against, *e.g*., at a distance from the mucosal surface that allows acceptably accurate measurement of carbon dioxide by a carbon dioxide sensor.

The term "patient" as used herein means a mammalian subject, preferably a human subject, that has, is suspected of having, or is or may be susceptible to a condition associated with low blood flow, and thus perfusion failure.

The present invention is based on applicants' discovery that increases in tissue CO₂ occur throughout the body during perfusion failure, rather than as only a localized phenomenon as previously believed in the art. The devices of the invention are thus designed to measure the partial pressure of CO₂ at a convenient site within the upper respiratory/digestive tract, and are thus performed in a minimally invasive manner. In general, the pCO₂ measurements are made by isolating an area of a mucosal surface at a selected site within the upper respiratory/digestive tract and using a sensor to detect pCO₂ at the selected site.

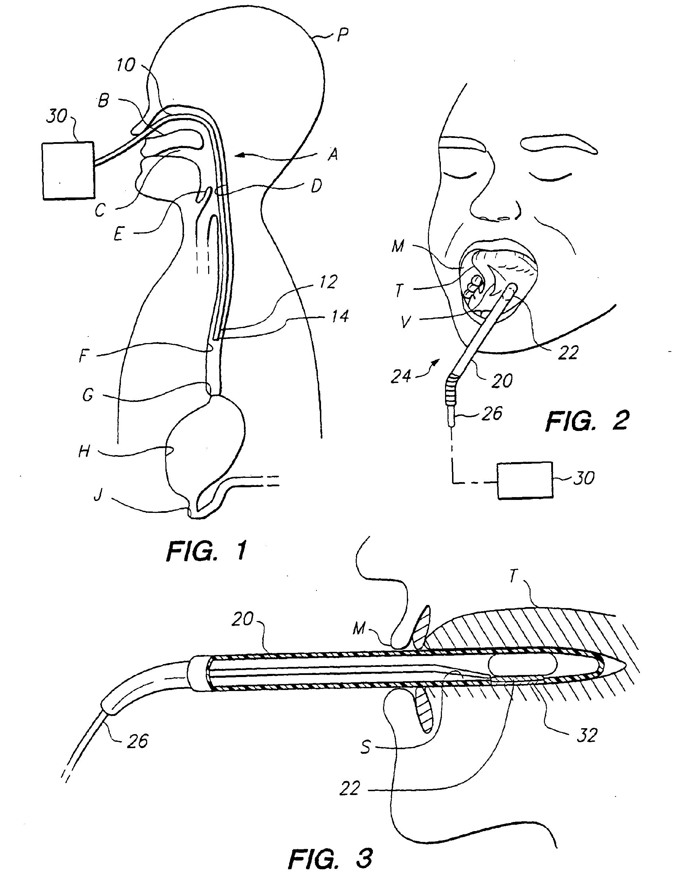

Fig. 1 illustrates the upper digestive/respiratory system or tract **A** of a person, and particularly including the nasal passage **B**. the oral passage **C**. and the upper portion **D** of the throat that extends to the top of the epiglottis **E**. The lower digestive (or gastrointestinal) tract includes the esophagus **F,** the esophageal sphincter **G,** the stomach **H,** and the intestines **J.** As discussed above, applicant earlier found that an accurate assessment of perfusion failure can be obtained by measuring the pCO₂ in the esophagus of a patient. These measurements involved the insertion of a catheter **10** (Fig. 1) with a CO₂ sensor **12** at the end, through the nasal or oral passage **B, C,** past the epiglottis **E**. and into the esophagus **F.** The end **14** of the catheter with the sensor **12** thereat, both lay within the esophagus. This procedure was advantageous in that the procedure's invasiveness was reduced and CO₂ generated by digestive fluids in the stomach did not affect measurements since the esophageal sphincter blocks such gas. However, the insertion of the catheter past the epiglottis **E** and into the esophagus, still constituted considerable invasion. In addition to harm that might be caused by threading the catheter into place, the fact that the catheter extended past the epiglottis **E** meant that the patient would also be exposed to the risk of regurgitation of stomach contents including stomach acids.

In accordance with the present invention, applicant finds that a highly useful measurement of perfusion failure can be obtained by measuring CO₂ in the upper digestive/respiratory tract **Ab**. with the sensor lying above, at the surface of, or at the epiglottis **E** so it does not have to pass by it. Preferably, the sensor is placed at a site within the oral-nasal cavity, *e.g*., within a nasal cavity, the mouth (*e.g*., under the tongue at a site in contact with the tongue or the floor of the mouth, between a region of the lip and gum or the cheek and gum, the roof of the mouth, or the soft palate), or the nasopharynx. Most preferably, the sensor is placed at a site that will avoid the patient's gag reflex or otherwise minimize discomfort.

The CO₂ sensor lies adjacent a mucosal surface in the upper digestive/respiratory tract **A**. in order that it effectively measures CO₂ in the tissue. Since carbon dioxide can readily pass through mucosal surfaces. CO₂ generated by metabolic activity occurring in tissue below the mucosal surface that is not carried away by blood flow readily migrates through the mucosal surface. Placement of a CO₂ sensor adjacent a mucosal surface of the upper digestive/respiratory tract **A** according to the present invention provides a very good quantification of perfusion failure at all times, including the most critical minutes after the onset of perfusion failure when treatment is likely to be most effective.

Fig. 2 shows one embodiment of a device or apparatus of the present invention, wherein a tube **20** containing a CO₂ sensor **22** at its front end, is inserted into the oral passage and placed under the tongue **T** of the patient, preferably to one side of the frenulum **V**. After insertion, it would be desirable if the mouth **M** of the patient is kept closed around the tube, so air does not circulate around the CO₂ sensor, which carries away some carbon dioxide. However, as with other instruments commonly inserted through the mouth, and as with a patient in a critical condition, the patient is usually unable to keep his mouth closed. Also, when the patient breathes through his nose, there is some air flow around the mouth. In such cases the device can be adapted with a holder as described below.

As illustrated in Fig. 2, the tube **20** and sensor **22** are part of an instrument **24** that includes a flexible cable **26** that extends to a test instrument **30** that typically indicates the partial pressure of CO₂, in millimeters of mercury (mmHg), which provides an indicia of a degree of perfusion failure. While the tube **20** is substantially rigid, the cable **26** is flexible. The cable **26** can be made highly flexible for ease of use, instead of having only the moderate flexibility of a catheter. Usually catheters require enough flexibility to pass through curved body passages, but yet must be resistant to column-type collapse in order to withstand the force applied to the catheter's proximal end necessary to accomplish insertion of the distal end and movement of the distal end along the body passage. Since the cable **26** in the device of Fig. 2 does not have to be pushed, it can have more flexibility for ease of use. The largely rigid tube **20** preferably has a length of no more than about one foot (one-third meter), since a longer length would be cumbersome. Catheters for insertion through the esophagus into the stomach, generally have a length of much more than two feet (0.61 m). Fig. 3 shows an' example of a sensor **22,** which lies against a membrane **32** which is in contact with the sublingual mucosal surface.

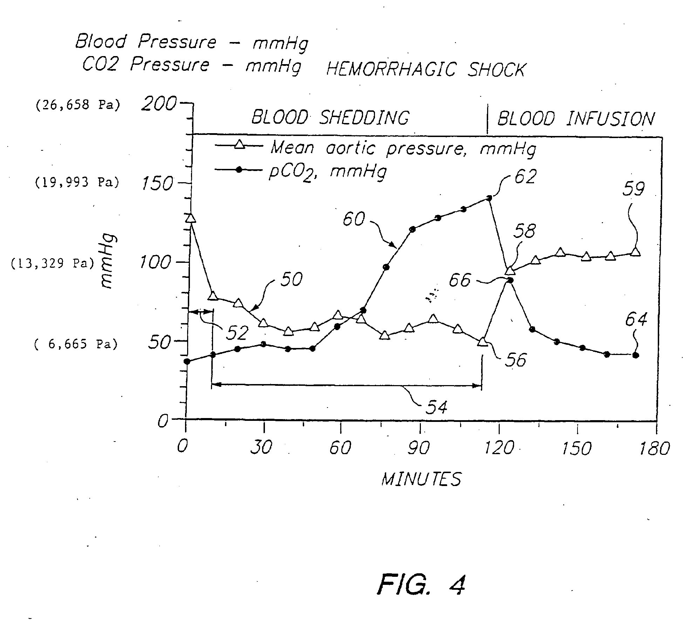

The correlation of perfusion failure with a increase in sublingual pCO₂, as well as the correlation of perfusion recovery and a decrease in sublingual pCO₂ was tested in an animal model that simulates a sudden loss or shedding of blood, such as might be caused by a gunshot wound or other severe wound. Perfusion recovery was simulated by subsequently reperfusing the animal with a blood infusion. The results are shown in Fig. 4. Graph line **50** (open triangles) is a measure of mean aortic pressure in mmHg throughout the test. Graph line **60** (closed circles) is a measure of sublingual pCO₂ obtained by a sensor.

At the beginning of the test (minutes = 0), considerable blood was drawn from an animal that was previously in good health, the blood being drawn within a period of a few minutes. Graph portion **52** of graph line **50** shows that aortic pressure rapidly dropped about 30% during the first few minutes of test. In a subsequent period **54** of about two hours, the aortic pressure remained about 40% below normal. The graph **60,** which shows that sublingual pCO₂ increased about 35% during the first **30** minutes, while aortic pressure **50** decreased by about 40%. From about 50 minutes to about 120 minutes, pCO₂ increased rapidly until the pCO₂ had increased by 300% above its initial value, as indicated by graph point **62.** These data show that an increase in sublingual pCO₂ is inversely correlated with aortic pressure during perfusion failure.

The relationship of pCO₂ and aortic pressure during perfusion recovery was tested by infusing the animal with a blood infusion at 120 minutes. The animal's aortic pressure rapidly increased, as shown by graph points **56** and **58,** until aortic pressure was restored to about 90% of original pressure before the test, as shown at graph point **59.** Sublingual pCO₂ rapidly decreased from point **62,** which was 300% above normal, to point **64,** which was only 25% above normal.

The results in the animal model can be extrapolated to represent a human subject suffering perfusion failure, such as that associated with a gunshot wound or a severe cut from machinery or a knife. The graph **50** thus illustrates that aortic pressure rapidly decreases during blood loss, until the outflow of blood is stopped by application of pressure or other means to stop bleeding. The present invention takes advantage of these phenomena to provide methods and devices to assist a physician or other health care provider in the diagnosis and treatment of a patient having or susceptible to a condition associated with perfusion failure.

For example, although assistance from a paramedic or other person may be available shortly after the initial primary insult, it may take thirty minutes or more for the patient to reach a hospital. This lapse in time may make it difficult to accurately assess the condition of the patient and the presence and/or severity of perfusion failure. Measuring and/or monitoring sublingual pCO₂ according to the present invention allows the physician or other healthcare provider to readily detect the level of pCO₂ relative to normal, as well as the rate of change of pCO₂. A rapid increase in pCO₂ suggests that the patient has suffered a loss of blood within the last hour or so, while a high level of pCO₂ indicates the patient presently suffers from a low level of aortic pressure and perfusion failure. In this manner the invention can be used to assess the patient's condition, allowing for appropriate and rapid selection of an appropriate therapy.

The present invention can also be used to monitor the efficacy of reperfusion or other therapeutic regimen to treat perfusion failure in the patient. For example, if the physician, paramedic, or other emergency provider determines that a transfusion of blood or blood components is indicated, and the transfusion is successful in rapidly increasing aortic pressure (such as that illustrated in Fig. 4 from graph points **56** to **58),** then this success will be reflected by a rapid drop in pCO₂ (as illustrated in Fig. 4 from graph points **62** to **66).** It is noted that the aortic pressure increases only moderately following this rapid rise until it stabilizes: in contrast, stabilization of pCO₂ is slightly delayed. This delay in pCO₂ stabilization is likely due to a delay in the removal of CO₂ at the site by the increased blood flow. Fig. 4 shows that sublingual measurement of pCO₂ provides a good indication of the level of perfusion failure.

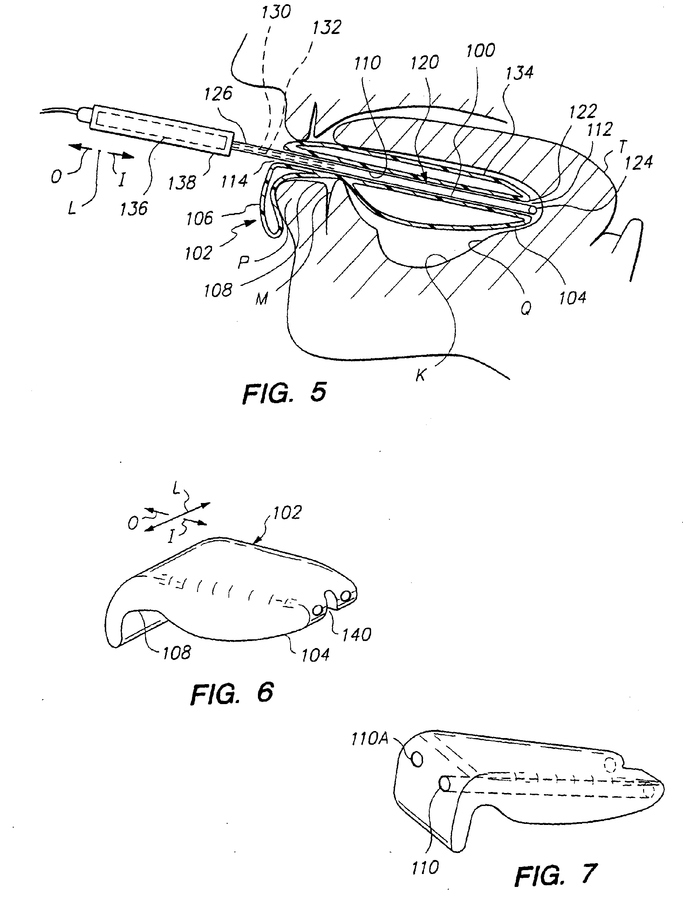

Fig. 5 shows a preferred embodiment of the device of the invention that is suitable for taking sublingual pCO₂ measurements. In this embodiment, sensor assembly instrument **100** is held in position by a sensor holder **102** that lies primarily in a patient's mouth. The sensor holder has a sublingual inner portion **104** that is shaped to fit under the patient's tongue **T,** and especially near the location where the tongue merges with the bottom or floor **K** of the mouth, and to lie on the bottom of the mouth. The holder has an outer portion **106** that lies outward of the inner portion and that is accessible from outside the mouth. The particular outer portion **106** lies outside the mouth and has a laterally **(L)** extending groove or recess **108** with groove walls that rest on the lower denture **M** and lower lip **P** of the patient.

The holder **102** forms a holder passage **110** that extends between the inner and outer portions **104, 106** of the holder. The passage has at least inner and outer ports **112, 114** and preferably extends along the entire length of the holder in the inner and outer directions I, O. The sensor assembly **100** has a frame **120** with an inner end **122** that supports a CO₂ sensor **124.** The sensor **124** projects inwardly from the holder and substantially directly contacts the mucosal surface Q of the patient. The frame has an outer end **126** that lies outside the patient's mouth. Where required for use with the CO₂ sensor, a pair of electrical conductors or wires 130. 132 may extend in the frame along the length of the passage between the sensor and an electrical circuit portion **136** mounted in a handle **138,** the circuit portion 136 preferably being a preamplifier but possibly being only a connector.

The holder **102** can serve at least two purposes. First the holder acts as an isolating means to isolate the mucosal surface area at and immediately around (for example, within about a centimeter or two) the measurement site (*e.g*., the location where the sensor touches the mucosal surface Q) from air flows in the mouth. Air flows around the sensor can sweep away some of the **CO₂,** resulting in an inaccurate reading. Furthermore, such isolation can also serve to trap moisture from the mucosal surface or from a device that adds moisture to the area where measurements are taken, thus decreasing any complications or measurement inaccuracies that may be associated with the sensor becoming too dry. To this end, the sublingual inner portion **104** of the holder preferably lies close to the walls of the mouth on opposite sides of the sensor **124,** as well as above and below the sensor. The upper surface **134** of the holder is designed so the tongue T can lie on at least its inner portion, to further provide a seal and to support the tongue to avoid tiring the patient.

While the holder is an exemplary and preferred isolating means for use with the present invention, other isolating means that serve substantially the same function can be substituted or used in conjunction with the holder. For example, a sheath can surrounds the CO₂ sensor, where the sheath contacts the mucosal surface around the perimeter of the sensor, thereby isolating the sensor from air flow. The sensor and the sheath can be held in place by a holder similar to that described above, but with the advantage that the entire device may be of an overall smaller size (*e.g*., for placement in the mouth).

A second purpose of the holder is to substantially fix the position of the sensor assembly **100** and the sensor **124** so the sensor does not move during an extended period of many minutes or even hours while the CO₂ of the patient is being measured. A tension coil spring extending between the handle and holder, can be used to gently urge the frame **120** inwardly, where necessary. The holder **102** is preferably formed of an elastomeric material (Young's modulus of less than 50,000 psi (3,446.5 x 10⁵ Pa)) such as a soft rubber or soft foam, to avoid high localized pressure on the patient's mouth that could discomfort him or her.

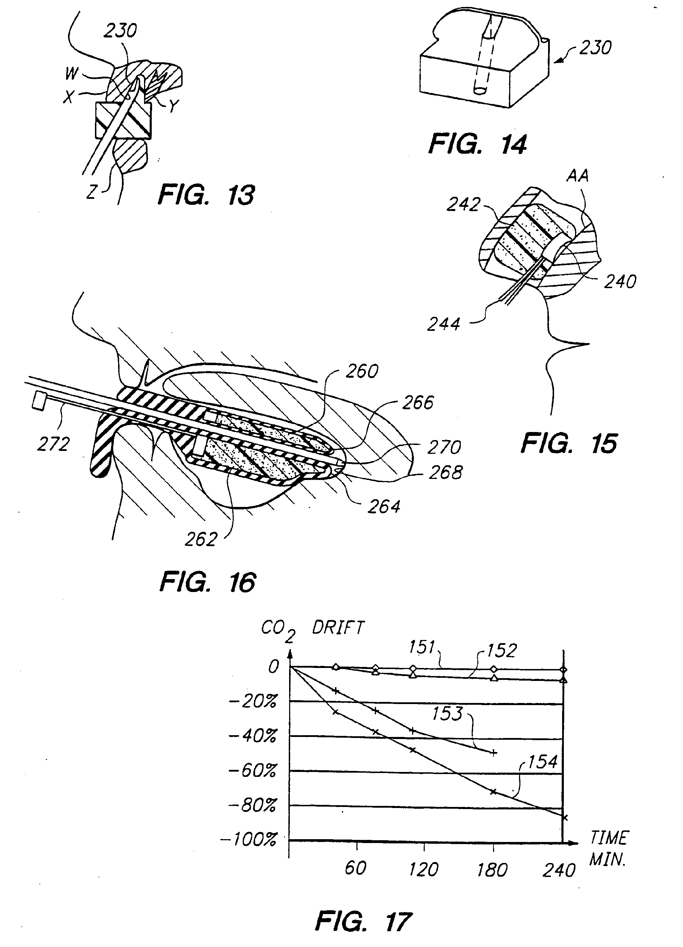

A third, optional purpose of the holder is to prevent or slow the drying out of the CO₂ sensor. As observed during extended duration tests performed by applicant, CO₂ sensors tend to dry out. Drying out of the sensor can be associated with false readings that indicate a lower CO₂ level than is actually present. Fig. 17 shows CO₂ drift when sensors were placed in different environments during tests. Graph lines **151, 152, 153,** and **154** respectively represent an environment of a 0.2% salt solution, human saliva, rat saliva, and air. The most drift was observed when the CO₂ sensor was used in air with substantially no isolation or added moisture.

The holder can be used to avoid drying out of the CO₂ sensor by isolating the sensor from air flow as discussed above. The holder can also be modified to add moisture to the area where measurements are taken. It should be noted that the CO₂ sensor may be used according to the present invention without a holder or humidification in the triage of a fully alert patient for a period of about one to two minutes. However, where the CO₂ sensor may be used in manner that renders the sensor susceptible to drying out, it is preferable to use the CO₂ sensor with a holder and/or to provide moisture to the site of measurement.

Preferably, the sensor is positioned on either side of the frenulum of the tongue. As shown in Figs. 6 and 7, the holder 102 is thus preferably formed with a slot 140 that receives the frenulum, so the sublingual inner portion **104** can lie close to the inner end of the sublingual area and therefore closely around the CO₂ sensor. The particular holder shown has two passages **110, 110A** that lead to areas on opposite sides of the frenulum. A thermometer can be inserted through the second passage, as the level of CO₂ is slightly affected by the patient's temperature. A thermometer can be incorporated in the instrument that includes the carbon dioxide sensor.

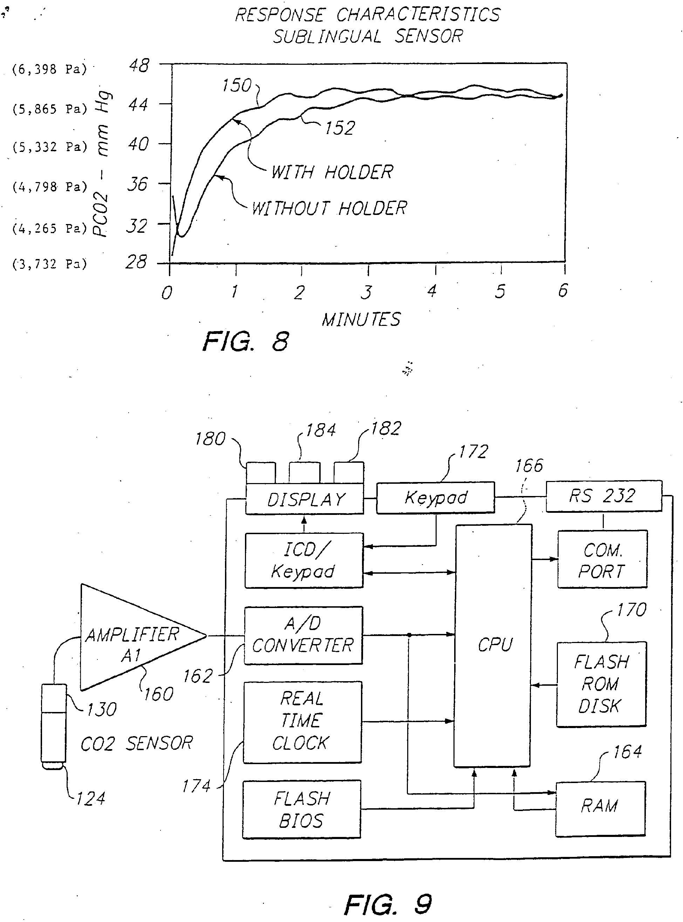

The importance of isolation of the sensor by, for example, use of the holder exemplified above, was tested in a healthy human volunteer who kept his mouth closed (around the holder and instrument) throughout the test, breathing only through his nose. The results are presented in the graph of Fig. 8, which shows pCO₂ (mmHg) versus time (minutes) with **150** and without **152** the holder **102.** With either arrangement, it can take a few minutes for the sensed level of CO₂ to reach a steady state. When the holder was used, the sensed level of CO₂ achieved steady state after about two minutes (graph line **150).** In contrast, steady state was achieved after about three minutes without the holder (graph line **152).** Furthermore, the measured level of CO₂ was somewhat higher with the holder than without the holder. These results suggest that use of the holder resulted in a decrease in removal of CO₂ from the mucosal surface engaged by the sensor. Because an ill patient might not keep his mouth closed, the air flow past the sensor would be greater than in the present experiment in which the subject kept his mouth closed. In such patients, the use of the holder may prove even more important in providing sensitive, accurate, and rapid CO₂ measurements.

The data provided by the CO₂ sensor may be acquired and analyzed by any appropriate means available. For example, Fig. 9 shows data acquisition circuitry that can be used to facilitate CO₂ data analysis. The circuit includes preamplifier **130** and amplifier **160,** which deliver signals representing the CO₂ level to an A/D converter **162.** The converter output is delivered to a memory **164** which stores the values and delivers them to a CPU, or central processing unit **166.** Software for instructing the CPU to operate on the data, is contained in a memory disk **170.** Pertinent information such as characteristics of the patient can be inputted through a keyboard **172.** CO₂ levels are delivered to the CPU at a rate of five samples per second. The CPU uses this data and the elapsed time from a clock **174** to deliver signals indicating the perfusion state of the patient. If the patient's condition is poor, a red light **180** is illuminated, if the patient's condition is stable a green light **182** is illuminated, and if the patient's condition is guarded a yellow light is illuminated **184.** This simplistic output is useful for moderately skilled persons such as medics in the armed forces and paramedics on ambulances. An indication of the patient's condition enables the health worker to determine whether or not the patient should be rushed to a treatment center and/or whether certain steps should be taken to enhance perfusion such as repeated depression of the chest.

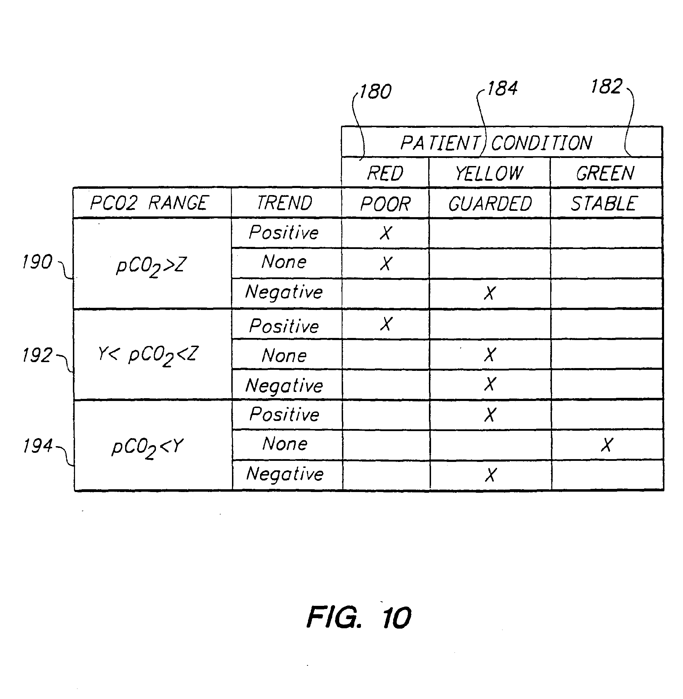

The software that controls the CPU can be programmed to operate on basic principles, such as those illustrated in Fig. 10. to determine which of the three signals (red light, green light or yellow light) should be displayed. In general, a particular high level of carbon dioxide **Z**. as well as a low level of carbon dioxide **Y** are established. These high and low levels may be, for example, Z = 80 mmHg (10.667 Pa) and Y = 50 mmHg (6,665 Pa). In addition, the CPU continually determines the rate of increase or decrease of pCO₂ For example, a rate of pCO₂ increase of more than 20 mmHa/hr. (2,666 Pa/hr.) will have a very negative implication for the patient. In comparison, a rate of pCO₂ increase less than 20 mmHg/hr. (2,666 Pa/hr.) has moderately negative or neutral implications for the patient. If the pCO₂ level is decreasing, or negative, this is usually positive.

As illustrated in the chart of Fig. 10, patients having a pCO₂ greater than **Z** are assigned to a first patient category **190.** If the rate of change of pCO₂ in these first category patients is zero or positive, then the condition of the patient is assessed as being poor and the red light at **180** is energized. If the pCO₂ is decreasing, then the yellow light **184** is energized to indicate that the patient is in a guarded state. If the initial pCO₂ measurement is between the two levels Z and Y, then the patient is assigned to a second patient category **192.** The condition of a second category patient is guarded, and thus the yellow light energized, unless the pCO₂ level is increasing at more than 20 mmHg/hr. (2,666 Pa/hr.), in which case the red light is energized. For a third patient category 194, the carbon dioxide level is less than **Y,** and the patient is deemed to be in a stable condition. If there is a considerable change in carbon dioxide, *e.g*., the CO₂ level increases at a rate of more than 20 mmHg/hr. (2,666 Pa/hr.) or decreases at a certain rate such as 10 mmHg/hr. (1,333 Pa/hr.) Where the CO₂ level is less than **Y**, a considerable change in CO₂ level may indicate that the patient suffers from a condition associated with abnormally high blood flow.

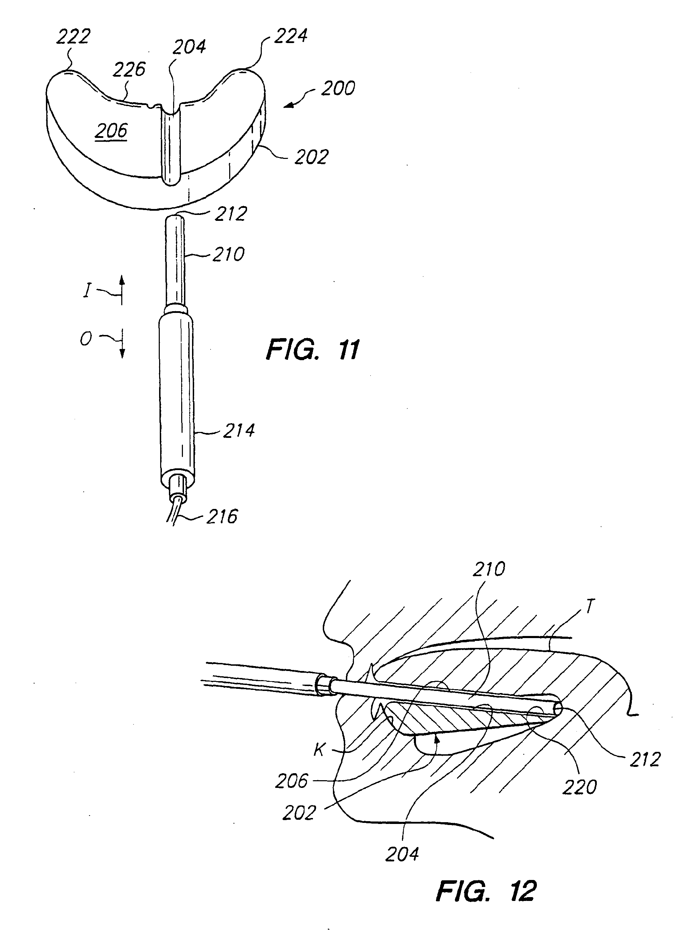

An additional exemplary holder useful with the present invention is illustrated in Figs. 11 and 12. The holder **200** basically includes a body **202** of plastic and preferably of elastomeric material, with an instrument passing passage in the form of a slot **204** in its upper surface **206**. A short rigid tube **210** with a carbon dioxide sensor **212** can fit in the slot. A short rigid handle **214** extends outwardly from the tube, while a flexible cable **216** extends largely outwardly from the handle. The instrument is preferably not longer than about 1/3rd meter.

Fig. 12 shows the placement of the holder body **202** lying completely within a person's mouth. The body **202** rests on the mouth floor at **K** with the CO₂ sensor **212** lying adjacent a sublingual mucosal surface area **220.** The tongue **T** of the person lies on the body upper surface **206** and seals the area directly behind the tongue. The body has a pair of opposite sides **222. 224** (Fig. 11) that project inwardly slightly more than the middle **226** to seal the opposite sides of the sensed area **220.** The rest of the body seals the region under and outward of area **220.** Only the tube **210** passes between the lips. Where appropriate, the holder and sensor may be fixed together, as with wires embedded in the body.

Although applicant prefers to place the sensor in a sublingual area, the sensor can be placed within any region of the upper respiratory/digestive tract, most preferably adjacent a mucosal surface of the mouth or nose. For example, the sensor **230** can be placed at a mucosal surface **W** that lies between a lip **X** and the teeth **Y** of the patient (Fig. 13). The area at the rear of the upper or lower lips **X, Z** is a mucosal surface from which CO₂ is drawn by blood flow. Figs. 13 and 14 illustrate a holder **230** suitable for use at a mucosal surface adjacent a patient's lips. In this embodiment, holder **230** is preferably of soft elastomeric material such as an elastomeric solid or a foam, or even a viscous fluid in a flexible shell. The holder isolates the mucosal surface area contacted by the sensor from air flow, thus preventing movement of the sensor and maintaining close to 100% humidity.

In another embodiment, the sensor **240** lies adjacent a mucosal surface area AA in a nares (nostril) of a patient (Fig. 15). A foam plug **242** serves as a holder that holds the sensor to position it, and that prevents air flow around the sensor. The foam plug can maintain close to 100% humidity. Only a pair of electrical wires **244** extend from the sensor through the holder. Where the CO₂ sensor is a fiber optical sensor, the holder can be adapted accordingly so that only the optical fiber extends from the plug.

As discussed above, it may be desirable to modify the holder or other portion of the device of the invention so as to prevent the CO₂ sensor from drying out. Fig. 16 shows a modified holder **260** which includes a sponge **262** containing a 0.2% salt solution (in water). Holes **264, 266** allow the weak solution to pass into the area **268** that is isolated by the holder, and where a CO₂ sensor **270** lies adjacent a mucosal surface. A plunger **272** can be pushed to compress the sponge and introduce the weak salt solution to the area (volume) containing the sensor to prevent dry out. Instead, a tube can be used to pass water vapor into the area **268** from a humidifier.

The CO₂ sensor used in the devices of the invention may be any CO₂ sensor suitable for detection of CO₂ in the manner described herein. For example, the CO₂ sensors used in the examples herein operate by detecting a change in pH in a solution surrounding a sensor. Specifically, such sensors have a membrane that is permeable to CO₂, and that separates a sodium bicarbonate or carbonic acid (HCO₃) solution from the environment. A pH sensor in the device measures the pH of the sodium bicarbonate solution. Two exemplary CO₂ sensors of this type, manufactured by Microelectrode. Inc. and by Nihon Kohden (ISFET pCO₂ sensor), were used by applicant in the examples herein. These CO₂ sensors are particularly susceptible to drying out, since solution within the sensor device can evaporate through the membrane.

Alternatively, the CO₂ sensor may be an optical CO₂ sensor. Structures, properties, functions, and operational details of fiber optic chemical sensors can be found in U.S. Patent Nos. 4,577,109; 4,785,814; and 4,842,783, as well as in Seitz, "Chemical Sensors Based on Fiber Optics." Anal. Chem. 56(1):16A-34A (1984). Fiber optic sensors for monitoring CO₂ that may be suitable for use in the present invention include, but are not limited to, those described in U.S. Patent Nos. 4, 892, 383: 4,919,891. 5,006,314; 5,098,659; 5,280,548: and 5,330,718. Other exemplary fiber optic CO₂ sensors are described in Peterson et al. "Fiber Optic Sensors for Biomedical Applications, "Science 224(4645):123-127 (1984) and Vurek et al. "A Fiber Optic pCO2 Sensor," Annals Biomed. Engineer. 11:499-510 (1983).

An especially preferred optical fiber CO₂ sensor is the sensor described in U.S. Patent No. 5.714.121 ('121), which describes an optical CO₂ sensor and methods of manufacture of same. In general, the sensor of the '121 patent is composed of a single optical fiber having a distal tip and a proximal region for communication with a means for receiving a signal from the distal tip. Light of a predetermined wavelength is directed through the optical fiber towards the distal tip, and emitted fluorescent light returns along the fiber to be detected and converted to a CO₂ concentration value. A capsule, is composed of a CO₂-permeable silicone material, is arranged over the distal tip at a predetermined position. The capsule contains an indicator solution having a suitable pH-sensitive indicator component, generally a fluorescent dye, preferably a reference dye as well, and substantially no air. A sealing means provides a liquid-tight seal and affixes the capsule onto the distal tip. A particularly preferred system employs hydroxypyrene trisulfuric acid (HPTS) as the fluorescent dye, and a rhodamine dye as the analyte-insensitive reference dye.

Optical CO₂ sensors are generally used by contacting the distal end of the sensor with a mucosal surface as described herein. Light of a predetermined wavelength is directed from an external source, through the optical fiber, impinging distally on the encapsulated indicator composition. The intensity of the emitted fluorescent light returning along the fiber is directly related to the concentration of CO₂ in the sample, as a result of the pH-sensitive indicator material present at the fiber tip *(i.e..* the pH of the indicator solution is directly related to CO₂ concentration, as a result of carbonic acid formation). The emitted light is carried by the optical fiber to a device where it is detected and converted electronically to a CO₂ concentration value. The sensor may additionally have a reference dye present in the indicator composition. The intensity of the light emitted form the reference dye may be used to compensate, via rationing, the signal obtained from the indicator.

Thus, the invention provides a device for assessing perfusion failure, which methods may be performed rapidly, with little equipment set up, and with minimal or substantially no invasion, and thus minimal risk of harm to the patient and an improved probability of patient compliance. A method of using the device of the invention generally involves introducing a CO₂ sensor into the upper digestive/respiratory tract of a patient, without passing the sensor down beyond the epiglottis where a first major intrusion would have occurred. Furthermore, the method can be performed so as to avoid even triggering the gag reflex of the patient. Measurements of CO₂ are taken while the sensor is held adjacent a mucosal surface in the upper digestive/respiratory tract, such as a mucosal surface of the mouth or nose, for example the area under the tongue, an area between the upper or lower lip and the teeth, or an area in the nose. A holder prevents sensor movement, while isolating the sensor area from random air flow such as inspired and expired gases which may otherwise dilute the submucosal CO₂, and while maintaining high humidity. The invention is useful in a variety of settings, such as in triage in emergency and disaster settings, monitoring in anesthesia, intensive care, and other acute settings in which patients may have acute perfusion failure (shock).

It is to be understood that while the invention has been described in conjunction with the preferred specific embodiments thereof, that the foregoing description as well as the examples which follow are intended to illustrate and not limit the scope of the invention. Other aspects, advantages and modifications within the scope of the invention will be apparent to those skilled in the art to which the invention pertains.

## Claims

1. A device for assessing perfusion failure in a patient, the device comprising:
a carbon dioxide sensor (22) means for detecting a partial pressure of carbon dioxide, the sensor means being adapted for lying adjacent a mucosal surface of the upper respiratory/digestive tract (A) of a patient and measuring carbon dioxide at the mucosal surface;
an indicating means (30) operably connected to the sensor means, wherein the indicating means indicates a degree of perfusion failure of the patient associated with the detected partial pressure of carbon dioxide; and
an isolating means (102) for inhibiting air flow around a region surrounding the mucosal surface adjacent the sensor means.

2. The device of claim 1, wherein the isolating means is a holder, the holder being designed to fit within the mouth of the patient and hold the sensor in place adjacent the mucosal surface.

3. The device of claim 1, wherein the isolating means is a holder, the holder being designed to fit within a nares of the patient and hold the sensor in place adjacent the mucosal surface.

4. The device of claim 1, wherein the device further comprises a moisturizing means for supplying moisture to the mucosal surface adjacent the sensor.

5. The device of claim 1, wherein the sensor is a fiber optic carbon dioxide sensor.

## Patentansprüche

1. Gerät zum Feststellen von Störungen der Perfusion eines Patienten, wobei das Gerät umfasst:
ein Kohlendioxidsensormittel (22) zum Detektieren eines Partialdrucks von Kohlendioxid, wobei das Sensormittel angepasst ist, benachbart einer Schleimhautoberfläche des oberen Atmungs/Verdauungstrakts (A) eines Patienten zu liegen und Kohlendioxid an der Schleimhautoberfläche zu messen,
ein mit dem Sensormittel betriebsmäßig verbundenes Anzeigemittel (30), wobei das Anzeigemittel einen Grad einer Störung der Perfusion des Patienten anzeigt, welcher dem detektierten Partialdruck von Kohlendioxid zugeordnet ist, und
ein Isolationsmittel (102) zum Unterdrücken von Luftströmung um ein Gebiet, welches die Schleimhautoberfläche benachbart zu dem Sensormittel umgibt.

2. Gerät nach Anspruch 1, wobei das Isolationsmittel ein Halter ist, wobei der Halter ausgestaltet ist, in den Mund des Patienten zu passen und den Sensor benachbart der Schleimhautoberfläche am Platz zu halten.

3. Gerät nach Anspruch 1, wobei das Isolationsmittel ein Halter ist, wobei der Halter ausgestaltet ist, in eine Nasenhöhle des Patienten zu passen und den Sensor benachbart zu der Schleimhautoberfläche am Platz zu halten.

4. Gerät nach Anspruch 1, wobei das Gerät weiterhin ein Befeuchtungsmittel umfasst, um der Schleimhautoberfläche benachbart zu dem Sensor Feuchtigkeit zuzuführen.

5. Gerät nach Anspruch 1, wobei der Sensor ein faseroptischer Kohlendioxidsensor ist.

## Revendications

1. Dispositif pour évaluer une déficience de l'irrigation sanguine chez un patient, le dispositif comprenant :
un moyen de détection de dioxyde de carbone (22) pour détecter une pression partielle de dioxyde de carbone, le moyen de détection étant adapté pour reposer adjacent à une surface muqueuse du système respiratoire/digestif supérieur (A) d'un patient et mesurer le dioxyde de carbone au niveau de la surface muqueuse ;
un moyen indicateur (30) connecté, de manière opérationnelle au moyen de détection, dans lequel le moyen indicateur indique un degré de déficience de l'irrigation sanguine du patient associée à la pression partielle de dioxyde de carbone ; et
un moyen d'isolation (102) pour empêcher un écoulement d'air autour d'une région entourant la surface muqueuse adjacente au moyen de détection.

2. Dispositif selon la revendication 1, dans lequel le moyen d'isolation est un support, le support étant conçu pour s'adapter à l'intérieur de la bouche du patient et supporter le détecteur en position adjacente à la surface muqueuse.

3. Dispositif selon la revendication 1, dans lequel le moyen d'isolation est un support, le support étant conçu pour s'adapter à l'intérieur d'une narine du patient et supporter le détecteur en position adjacente à la surface muqueuse.

4. Dispositif selon la revendication 1, dans lequel le dispositif comprend en outre un moyen d'humidification pour délivrer de l'humidité à la surface muqueuse adjacente au détecteur.

5. Dispositif selon la revendication 1, dans lequel le détecteur est un détecteur de dioxyde de carbone à fibre optique.
